# EUROPEAN PATENT APPLICATION

(11) **EP 1 508 798 A1**
(43) Date of publication of application: **23.02.2005**
(21) Application number: 04019422.7
(22) Date of filing: 16.08.2004
(51) Int. Cl.: G01N 21/89, G01N 33/36, B65H 63/06

(54) **Detecting device for foreign material in yarn**

(30) Priority: 21.08.2003 JP 2003297007
(71) Applicant: MURATA KIKAI KABUSHIKI KAISHA, Minami-ku Kyoto-shi Kyoto 601 (JP)
(72) Inventor: Nakade, Kazuhiko, Kyoto-shi Kyoto (JP); Yoshikawa, Keiji, Yokohama-shi Kanagawa (JP)
(74) Representative: Liedl, Christine

(57) **Abstract**

The present invention accurately detects foreign material mixed into a yarn using a simple configuration. A yarn 1 is irradiated with ultraviolet rays alternately emitted by light sources UV1 and UV2. Light receiving elements PD1 and PD2 then alternately receive a light reflected by the yarn 1. Sample-and-hold circuits 3c and 3d then hold signals corresponding to the quantities of light received. An adder 4b then adds the signals for the quantities of light reflected together. Thus, the mixture of foreign material is detected on the basis of a change in the added signal (Fig.1).

## Description

### Field of the Invention

The present invention relates to a detecting device that detects foreign material mixed into a spun yarn.

### Background of the Invention

In a spinning machine that aggregates fibers such as cotton together and twists the aggregate into a yarn, a detecting device is placed which detects a defect in a yarn running from a spinning section to a winding bobbin. When a defect occurs in the yarn, it is detected by the detecting device and the yarn is cut. Then, the defective part is removed and the remaining parts are connected together by, for example, being twisted together. Thus, the yarn continues to be completed.

One of such known devices detecting a defect in the yarn detects the mixture of, for example, blackish or bright foreign fibers by irradiating the running yarn with light to receive light reflected by the yarn and transmitted through the yarn to detect the mixture of such foreign fibers on the basis of both amounts of light received. This device utilizes a phenomenon described below. The presence of a blackish foreign fiber slightly reduces both quantity of reflected light received and quantity of reflected light received, whereas the presence of a bright foreign fiber slightly increases both quantity of reflected light received and quantity of transmitted light received. The amount of change in the quantity of reflected light received is then added to the amount of change in the quantity of transmitted light received. The defect is thus detected on the basis of the result of the addition.

Such a detecting device is disclosed, for example, in the Japanese Patent Publication No. 3176923

In this case, foreign material with a different color can be detected by using a light source that generates a visible light (in general, a light source for a green color in an intermediate wavelength region of the visible light).

However, when defects caused by foreign material are detected by using a light source that generates a visible light, the quantity of reflected light received and the quantity of transmitted light received differ by depending on the thickness of the yarn. Accordingly, to detect such a defect as the increased thickness of the yarn resulting from the mixture of foreign material, it is necessary to add together the quantity of reflected light received and the quantity of transmitted light received in order to discriminate this defect from a simple defect in yarn thickness.

It is an object of the present invention to enable foreign material mixed into a yarn to be accurately detected using a simple configuration, to solve the above problems.

### Summary of the Invention

The present invention is mainly characterized by comprising floodlighting means for irradiating a running yarn with an ultraviolet ray and reflected light receiving means for receiving that part of the ultraviolet ray applied by the floodlighting means which is reflected by the yarn to output a signal corresponding to the quantity of light received, and in that foreign material mixed into the yarn is detected on the basis of the magnitude of the output signal from the reflected light receiving means.

The detecting device that detects foreign material in a yarn according to the present invention irradiates the running yarn with an ultraviolet ray to detect foreign material mixed into the yarn on the basis of the quantity of ultraviolet ray reflected by the yarn. The accuracy with which the foreign material is detected can thus be increased. The present detecting device also eliminates the need for a process of adding together the quantity of reflected light received and the quantity of transmitted light received, thus simplifying the configuration of the foreign material detecting device. With an ultraviolet ray, the quantity of light reflected changes very insignificantly in connection with the thickness of a yarn containing cotton fibers as a material. On the other hand, the quantity of light reflected changes significantly in the presence of homogeneous fibers in different colors or heterogeneous white objects such as white polypropylene films. Therefore, an accurate foreign material defect detecting device can be obtained simply by detecting the quantity of light reflected.

### Brief Description of the Drawings

Figure 1 is a diagram of a block circuit for a detecting device for foreign material in a yarn according to the present invention (Embodiment 1).
Figure 2 is a timing chart of the block circuit shown in the embodiment 1.
Figure 3 is a chart illustrating operations of the block circuit shown in the embodiment 1.
Figure 4 is a diagram showing the configuration of a detecting section in the embodiment 1.
Figure 5 is a diagram of a block circuit for a detecting device for foreign material in a yarn according to the present invention (Embodiment 2).
Figure 6 is a diagram showing another configuration of the detecting section according to Embodiment 2.
Figure 7 is a characteristic curve diagram showing the relationship between a reflected light signal and a transmitted light signal. Figure 7A indicates a green light source, and Figure 7B indicates a UV light source.
Figure 8 is a diagram showing a reflected light signal change rate for each light source.

### Detailed Description of the Preferred Embodiments

The object to accurately detect foreign material mixed into a yarn using a simple configuration was accomplished by irradiating the running yarn with an ultraviolet ray to receive a part of the ultraviolet ray which is reflected by the yarn.

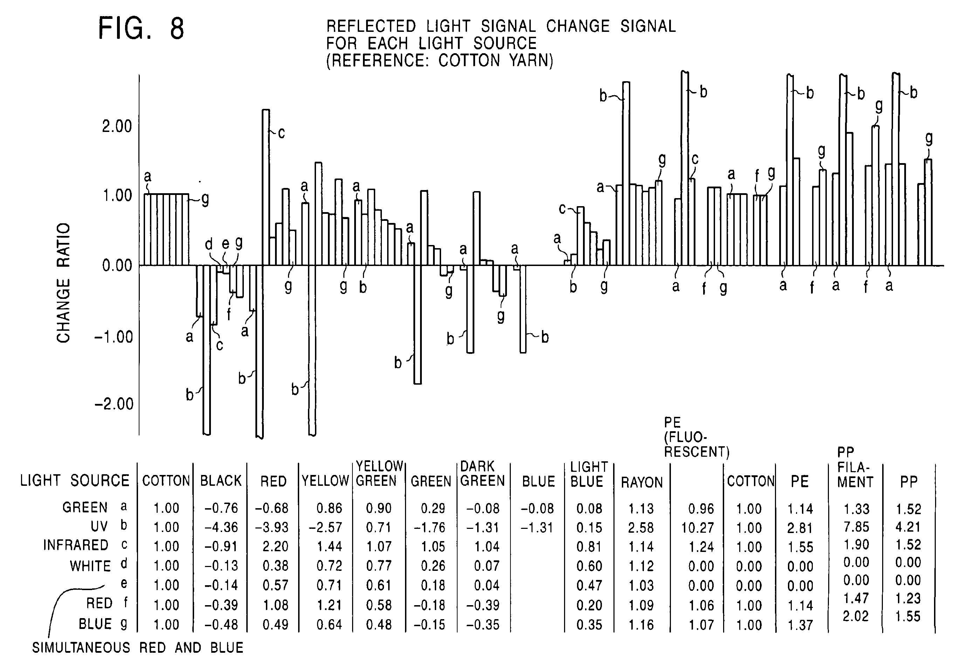

Figure 8 shows the results of examination of the change rate of the quantity of light reflected using different light sources for a green light (a), an ultraviolet light (b), an infrared light (c), a white light (d), a simultaneous blue and red light (e), a red light (f), and a blue light (g). In this case, the quantity of light reflected by a white cotton yarn was used as a reference to examine the change rate of the quantity of light reflected by colored yarns including a black yarn, a red yarn, a yellow yarn, a green yarn, a dark green yarn, a blue yarn, and a light blue yarn, and white synthetic fiber yarns including a rayon yarn, a polyester yarn (PE fluorescent), a falsely twisted polyester yarn (PE), and a yarn composed of white polypropylene (PP) filaments, and a white polypropylene film (PP).

As is apparent from Figure 8, the ultraviolet light (b) exhibits a higher change rate for each colored yarn than the other light sources. In particular, the change rate is high for the various white synthetic fiber yarns and white PP film, composed of materials different from cotton. That is, the ultraviolet light (b) enables foreign material mixed into the yarn to be accurately detected simply by detecting the quantity of light reflected by the yarn.

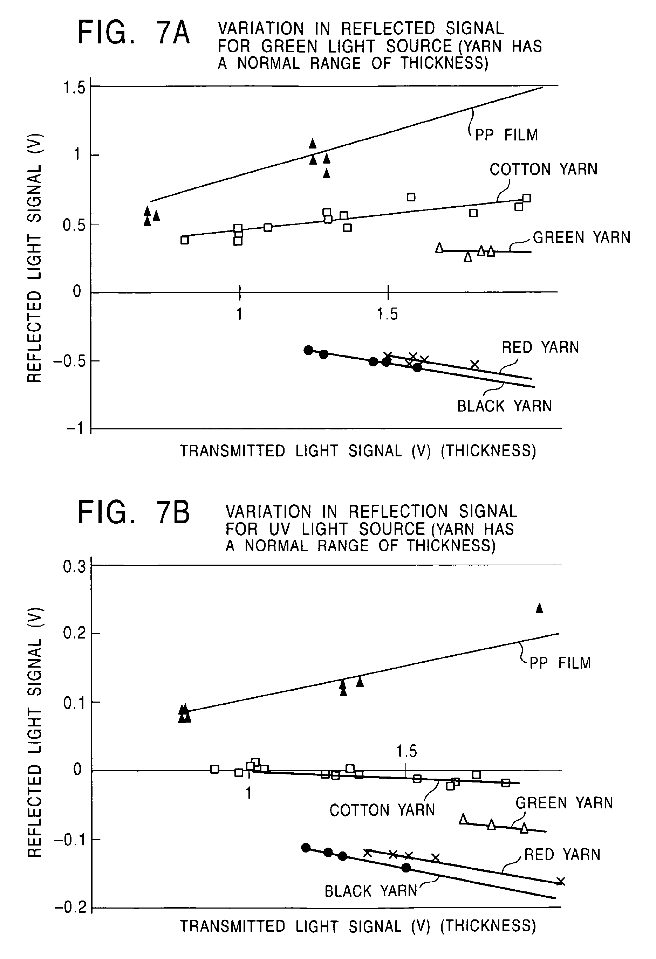

Figure 7A shows the results of examination of the relationship between the quantity of light (vertical axis) reflected by various yarns and the thickness of a target (horizontal axis) to be irradiated with light if a light source for a green color having an intermediate wavelength of a visible light is used to irradiate a white cotton yarn, a black yarn, a red yarn, a green yarn, and a PP film with the green light. Figure 7B shows the results of examination of the relationship between the quantity of light (vertical axis) reflected by various yarns and the thickness of a target (horizontal axis) to be irradiated with light if a light source generating an ultraviolet ray is used to irradiate a cotton yarn, a black yarn, a red yarn, a green yarn, and a PP film with the ultraviolet ray. It should be noted that for the convenience of measurements, the vertical axes in Figures 7A and 7B have different scales.

If the white cotton yarn (shown by white squares) is irradiated with the green light, the quantity of light reflected increases consistently with the quantity of light transmitted (thickness) as shown in Figure 7A. However, if the white cotton yarn is irradiated with the ultraviolet yarn, the quantity of light reflected is generally much smaller than that of light reflected if the yarn is irradiated with the green light as shown in Figure 7B. In this case, even with an increase in the quantity of light transmitted (thickness), the quantity of light reflected changes very insignificantly. Rather, the quantity of light reflected decreases slightly with increasing quantity of light transmitted (thickness).

If the PP film (shown by black triangles) is irradiated with the green light, the quantity of light reflected is about twice as large as that of light reflected by the white cotton yarn as shown in Figure 7A. However, for both PP film and white cotton yarn, the quantity of light reflected increases consistently with the quantity of light transmitted (thickness). This indicates that if the PP film is mixed into the white cotton yarn, then even with an increase in the quantity of light reflected, this change is almost the same as the change in thickness. It is thus impossible to discriminate the white cotton yarn mixed with the PP film from the same yarn without the PP film on the basis of only the quantity of light reflected.

On the other hand, if the PP film is irradiated with the ultraviolet ray, the quantity of light reflected is much larger (four or more times as large as) than that of light reflected by the white cotton yarn as shown in Figure 7B. Moreover, the quantity of light reflected by the white cotton yarn decreases very insignificantly compared to the change in thickness, and rather decreases. In contrast, the quantity of light reflected by the PP film increases in proportion to the quantity of light transmitted (thickness). This indicates that the quantity of light reflected increases in proportion to the amount of PP film mixed, regardless of the thickness of the white cotton yarn.

Specifically, if a PP film that is white like the white cotton yarn is mixed into an original yarn as foreign material, it is very difficult to detect the presence of the PP film by irradiation with the green light even using yarn thickness information in addition to a reflection signal. In contrast, with irradiation with the ultraviolet ray, the quantity of light reflected changes markedly in response the mixture of the PP film into the original yarn. Consequently, the mixture can be easily detected on the basis of only the change in the quantity of light reflected.

If the black yarn (shown by black circles) and the red yarn (shown by crosses) are irradiated with the green light, the quantity of light reflected is equal to the quantity of light reflected by the white cotton yarn multiplied by minus one. Accordingly, the light is absorbed but a change in the quantity of light is proportional to the thickness of the yarn as in the case of a change in the reflected light signal from the white cotton yarn. That is, if the black or red yarn is mixed into the white cotton yarn, the quantity of light reflected changes in proportion to the rate of mixture but is also affected by a change in thickness. Accordingly, the thickness of the yarn must be determined and this foreign material cannot be determined on the basis of only the quantity of light reflected. For the green yarn (shown by white triangles), the quantity of light reflected is about half that of light reflected by the white cotton yarn. The detection itself of the mixture is thus difficult. In contrast, if these yarns are irradiated with the ultraviolet ray, the quantity of light reflected by the black or red yarn is five or more times as large as that of light reflected by the white cotton yarn. The quantity of light reflected by the green yarn is three or more times as large as that of light reflected by the white cotton yarn. Consequently, the mixture can be detected on the basis of only the quantity of light reflected.

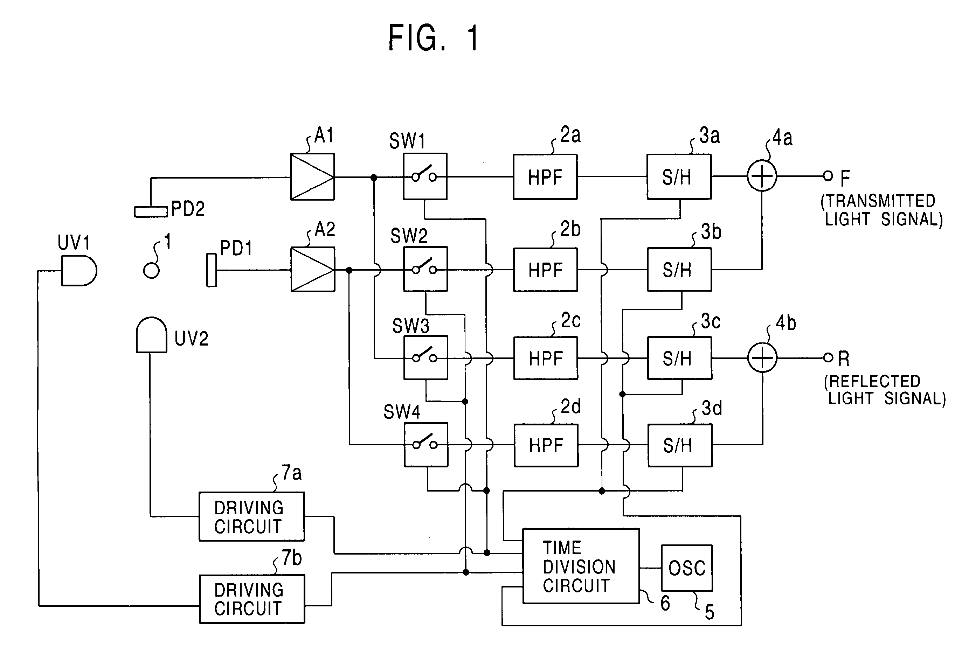

Figure 1 shows a block circuit for a device detecting foreign material in a yarn according to an embodiment of the present invention.

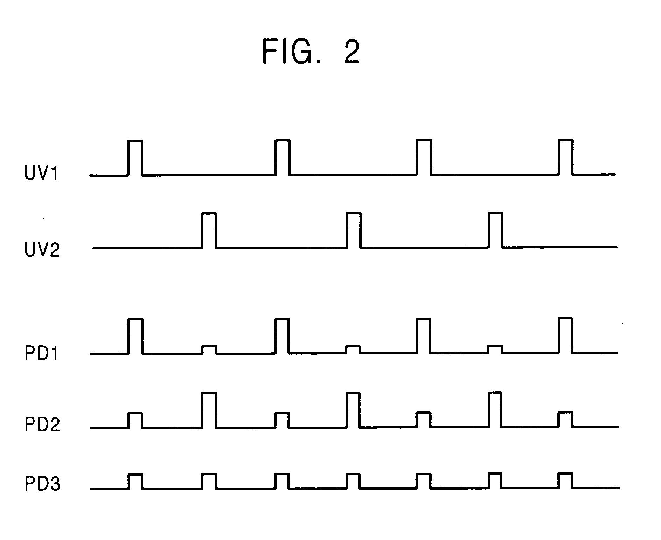

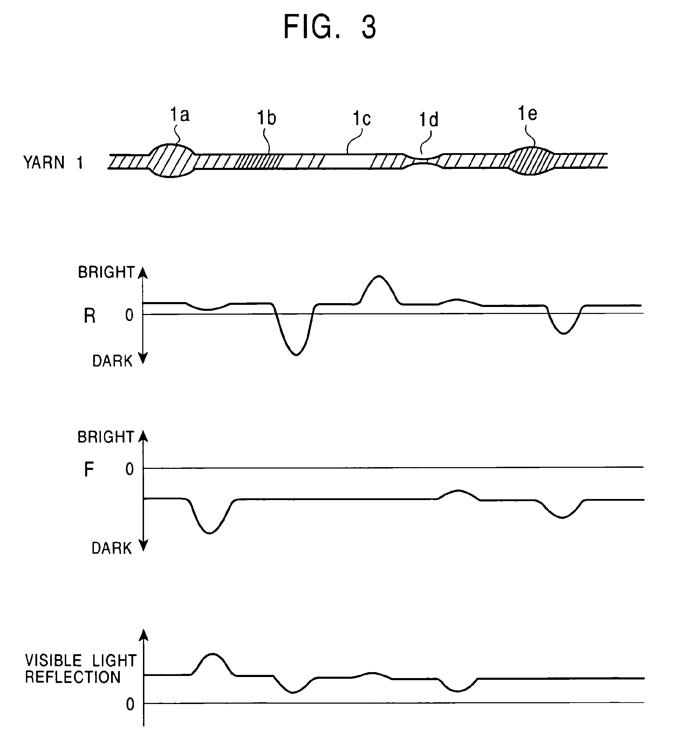

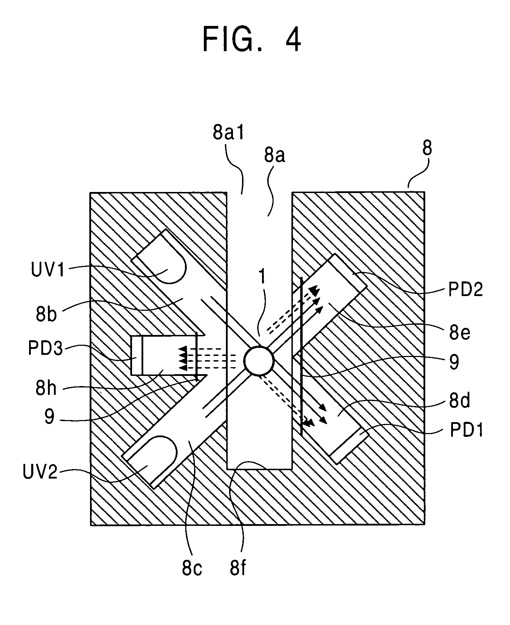

Figure 2 is a timing chart for the block circuit shown in Figure 1. Figure 3 illustrates operations of the block circuit shown in Figure 1. Figure 4 is a diagram showing the configuration of a detecting section (sensor head).

In Figure 1, 1 is a yarn, UV1 and UV2 are light sources such as LEDs which generate ultraviolet rays. PD1 and PD2 are light receiving elements, and A1 and A2 are amplifiers. SW1 to SW4 are switching elements, 2a to 2d are high pass filter circuits, and 3a to 3d are sample-and-hold circuits. Further, 4a to 4d are adders, 5 is a transmitter, 6 is a time division circuit, and 7a and 7b are UV light source driving circuits.

The yarn 1 is irradiated with lights from the light sources UV1 and UV2 in different directions. The light receiving element PD1 receives a transmitted light applied by the light source UV1 and transmitted through the yarn 1, and a reflected light applied by the light source UV2 and reflected by the yarn 1. The light receiving element PD2 receives a transmitted light applied by the light source UV2 and transmitted through the yarn 1, and a reflected light applied by the light source UV1 and reflected by the yarn 1.

The time division circuit 6 divides an output pulse from the transmitter 5 to simultaneously drive the UV light source driving circuit 7a, the switching elements SW1 and SW4, and the sample-and-hold circuits 3a and 3d, while simultaneously driving the UV light source driving circuit 7b, the switching elements SW2 and SW3, and the sample-and-hold circuits 3b and 3c; the time division circuit 6 alternately drives the former and latter groups. The UV light source driving circuit 7a is driven to cause the light source UV2 to generate an ultraviolet ray to irradiate the yarn 1 with the ultraviolet ray as shown in Figure 2. The light receiving element PD2 receives the ultraviolet ray transmitted through the yarn 1 to output a signal corresponding to the quantity of light received. The light receiving element PD1 receives the ultraviolet ray reflected by the yarn 1 to output a signal corresponding to the quantity of light received. At this time, the switching elements SW1 and SW4 are already closed (the switching elements SW2 and SW3 are open). The output signal corresponding to the quantity of transmitted light received by the light receiving element PD2 passes through the high pass filter circuit 2a and is held by the sample-and-hold circuit 3a. The output signal corresponding to the quantity of reflected light received by the light receiving element PD1 passes through the high pass filter circuit 2d and is held by the sample-and-hold circuit 3d.

Then, the UV light source driving circuit 7b is driven to cause the light source UV1 to generate an ultraviolet ray to irradiate the yarn 1 with the ultraviolet ray as shown in Figure 2. The light receiving element PD1 receives the ultraviolet ray transmitted through the yarn 1 to output a signal corresponding to the quantity of light received. The light receiving element PD2 receives the ultraviolet ray reflected by the yarn 1 to output a signal corresponding to the quantity of light received. At this time, the switching elements SW2 and SW3 are already closed, and the switching elements SW1 and SW4 are open. The output signal corresponding to the quantity of transmitted light received by the light receiving element PD1 passes through the high pass filter circuit 2b and is held by the sample-and-hold circuit 3b. The output signal corresponding to the quantity of reflected light received by the light receiving element PD2 passes through the high pass filter circuit 2c and is held by the sample-and-hold circuit 3c.

The adder 4a adds the signals held by the sample-and-hold circuits 3a and 3b together and outputs a transmitted light signal F. The adder 4b adds the signals held by the sample-and-hold circuits 3c and 3d together and outputs a reflected light signal R. The transmitted light signal F is a thickness signal for the yarn 1 and the reflected light signal R indicates whether or not foreign material is mixed into the yarn 1.

By driving the light sources UV1 and UV2, the switching elements, and the sample-and-hold circuits synchronously with an output pulse from the time division circuit 6, it is possible to prevent the averse effects of an externally invading light or the other light source (UV1 or UV2).

Moreover, the mixture of foreign material into the yarn 1 can be monitored from two different directions to more reliably detect foreign material, and the input signals from the two directions can be alternately inputted to the time division circuit 6 to enable foreign material to be generally continuously monitored.

Furthermore, the input signals from the two directions can be alternately inputted to the time division circuit 6 to enable the inputting of not only the reflected light but also the transmitted light.

The yarn 1 is assumed to have a thick portion 1a, a portion 1b into which dark foreign material has been mixed, a portion 1c into which bright foreign material has been mixed, a thin portion 1d, and a portion 1e into which thick and dark foreign material has been mixed, as shown in Figure 3. Then, as shown at R in Figure 3, the reflected light signal R varies insignificantly in the thick portions 1a and thin portions 1d, and significantly in the portions 1b, 1c, 1e into which foreign material has been mixed. Thus, the mixture of foreign material can be detected regardless of the thickness of the yarn 1.

As shown at F in Figure 3, the transmitted light signal F varies in proportion to the thickness in the thick portion 1a, the thin portion 1d, and the thick portion 1e into which foreign material has been mixed. No change occurs in the portions 1b or 1c even with the mixture. In the thickness varying portion 1d, the magnitude of the transmitted light signal F varies in proportion to the thickness. This makes it possible to detect a defect in the thickness of the yarn 1 regardless of the mixture of foreign material.

In contrast, with visible light, as shown by the visible light reflection in Figure 3, the magnitude of the reflected light signal decreases in the portion 1b into which black foreign material has been mixed, and in the thin portion 1d. Accordingly, the reflected light signal alone cannot discriminate the portions 1b and 1c from each other. Further, the magnitude of the signal increases both in the thick portion 1a, and the portion 1c into which bright foreign material has been mixed. Even if the black foreign material is mixed into the yarn 1, the change in the signal from the thick portion 1e varies depending on the mixture rate and thickness, and in some cases, the signal does not substantially change. In any case, foreign material cannot be detected on the basis of only the reflected light signal. Thus, for example, the reflected light signal must be added to the transmitted light signal.

In this embodiment, the reflected light receiving means for outputting a signal for the ultraviolet ray reflected by the yarn 1 is composed of a circuit composing of the light receiving elements PD1 and PD2, the amplifiers A1 and A2, the switching elements SW3 and SW4, the high pass filter circuits 2c and 2d, the sample-and-hold circuits 3c and 3d, and the adder 4b. However, the reflected light receiving means may be further provided with a light receiving element PD3 that receives only the light applied by the light sources UV1 and UV2, and reflected by the yarn 1. PD3 in Figure 2 is operation timings in this case.

Figure 4 shows the light sources UV1 and UV2 which generate ultraviolet rays, and the light receiving elements PD1 and PD2 which receive the ultraviolet rays from the light sources UV1 and UV2. The light sources and the light receiving elements are housed in one case 8. The case 8 comprises a passage 8a used as a passage space through which the yarn 1 is passed, an opening 8a1 through which the yarn 1 can be inserted into the passage 8a, a hollow chamber 8b in which the light source UV1 is installed, a hollow chamber 8c in which the light source UV2 is installed, a hollow chamber 8d in which the light receiving element PD1 is installed, and a hollow chamber 8e in which the light receiving element PD2 is installed. Further, 8h is a hollow chamber in which the light receiving element PD3 receiving only the reflected light is installed if the light receiving element PD3 is provided.

The hollow chambers 8b, 8c, 8d, 8e, 8h are open toward the yarn 1. A visible light cut filter 9 is provided at each of the openings in the hollow chambers 8d, 8e, 8h. At least a bottom surface 8f of the passage 8a, that is, the surface of the passage 8a which is opposite the opening 8a1 is black. This hinders light entering through the opening 8a1 from being reflected by an inner wall surface defining the passage space. At the same time, the light receiving element PD1 receives only an ultraviolet ray emitted by the light source UV1 and transmitted through the yarn 1. The light receiving element PD2 receives only an ultraviolet ray reflected by the yarn 1. The light receiving element PD2 receives only an ultraviolet ray emitted by the light source UV2 and transmitted through the yarn 1. The light receiving element PD1 receives only an ultraviolet ray reflected by the yarn 1. The light receiving element PD3 receives only a light applied by the light sources UV1 and UV2 and reflected by the yarn 1.

The above-mentioned embodiment 1 comprises a circuit that forms a transmitted light signal F so as to be able to detect a defect in the thickness of the yarn. However, if it is sufficient to detect a foreign material mixture defect, this circuit may be omitted. In this case, one of the two light sources applying an ultraviolet ray may be omitted. Alternatively, the time division circuit may be omitted.

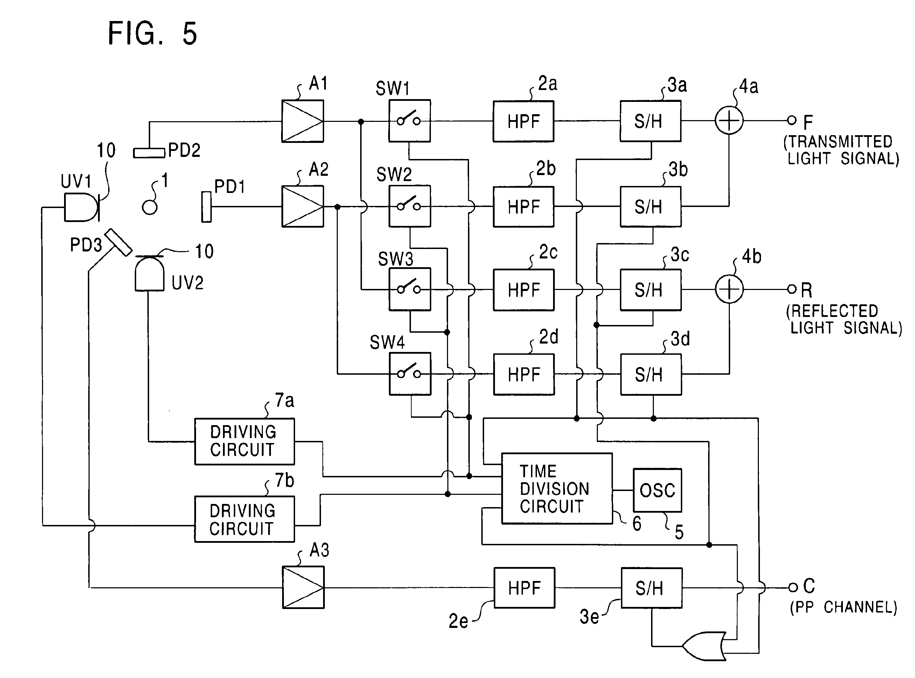

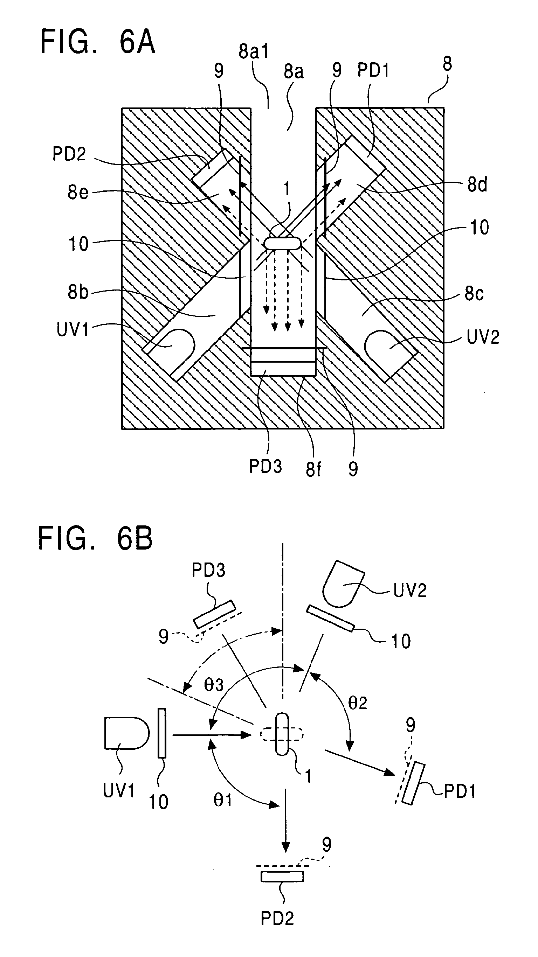

Figure 5 shows a block circuit for a device detecting a defect in a yarn according to another embodiment of the present invention, that is, the embodiment shown in Figure 1 wherein the light receiving element PD3 is further provided to receive only the lights applied by the light sources UV1 and UV2 and reflected by the yarn 1. Figure 6 is a diagram showing the configuration of a detecting section (sensor head). Parts of this embodiment corresponding to those of Embodiment 1, shown in Figures 1 to 3, are denoted by the same reference numerals, and their detailed description is omitted. With this embodiment, if a PP film which is a packing material used to pack cotton fibers or the like, a material for spun yarns, is mixed into the yarn during a mixing and blowing step, and the PP film fails to be twisted to make the yarn flat, it is possible to more reliably detect the flat yarn defect caused by the mixture of the foreign material.

It may be difficult to detect such a flat yarn defect using the two light sources UV1 and UV2, and two light receiving elements PD1 and PD2 as shown in Embodiment 1. Thus, in this embodiment, the light receiving element PD3 is added to enable the detection of a flat yarn detect regardless of the direction of the flat portion.

Specifically, the block circuit shown in Figure 5 is the block circuit shown in Figure 1 to which the following components are added: the light receiving element PD3 which receives those parts of the ultraviolet rays applied by the light sources UV1 and UV2 to the yarn 1 which are reflected by the yarn 1, an amplifier A3 that amplifies an output signal from the light receiving element PD3, a high pass filter circuit 2f, and a sample-and-hold circuit 3e. The sample-and-hold circuit 3e holds a detection signal C for the above defect.

The sample-and-hold circuit 3e is driven by every pulse signal outputted by the time division circuit 6. The sample-and-hold circuit 3e uses the operation timings shown at PD3 in Figure 2 to hold the output signal for the quantity of received light applied by the light sources UV1 and UV2 to the yarn 1, reflected by the yarn 1, and received by the light receiving element PD3.

The light sources UV1 and UV2, and the light receiving elements PD1, PD2, and PD3, which receive the ultraviolet rays from the light sources UV1 and UV2, may be configured as shown in Figure 4. However, the light receiving element PD3 that exclusively receives the reflected light may be installed on the bottom surface 8f of the passage 8a as shown in Figure 6A. In Figure 6A, parts of this embodiment corresponding to those of the configuration shown in Figure 4 are denoted by the same reference numerals, and their detailed description is omitted.

Reference numeral 10 shown in Figure 6A is a diffuser panel that diffuses ultraviolet rays applied by the light sources UV1 and UV2. The diffuser plate 10 diffuses ultraviolet rays applied by the light sources UV1 and UV2, in all directions to irradiate the yarn 1 with the ultraviolet rays. Then, the light receiving elements PD1, PD2, and PD3 receives the diffused reflected lights.

In the arrangements shown in Figures 4 and 6A, the light sources UV1 and UV2 are arranged on a plane orthogonal to the running yarn 1 and away from each other by about 90 degrees around the running yarn 1. The light receiving element PD1 is located opposite the light source UV1 across the yarn 1. The light receiving element PD2 is located opposite the light source UV2 across the yarn 1. The light receiving element PD3 is located halfway between the light sources UV1 and UV2, arranged away from each other by about 90 degrees.

This arrangement enables the light receiving element PD1 to receive a light from the light source UV1 transmitted through the yarn 1 and a light from the light source UV2 reflected by the yarn 1 in a 90 degrees direction with respect to the incident light. The light receiving element PD2 receives a light from the light source UV2 transmitted through the yarn 1 and a light from the light source UV1 reflected by the yarn 1 in the direction opposite to the 90 degrees one. The light receiving element PD3 can receive lights from the light sources UV1 and UV2 which are reflected by the yarn 1 in the direction halfway between the light receiving elements PD1 and PD2. That is, a flat yarn defect can be detected over a wide angle range within which the flat portion appears.

However, with this arrangement, if foreign material lies at a detected position, for example, with its flat sectional shape extending over the optical axis of the light source UV1, a light from the light source UV2 is reflected toward the light source UV2. Consequently, the light receiving element PD1 cannot detect the reflected light. Further, the light receiving surface of the light receiving element PD2 is opposite the surface of the flat yarn 1. Accordingly, an ultraviolet ray applied by the light source UV1 to the yarn 1 is applied to the thin surface of the yarn 1. Thus, the light may not be reflected toward the light receiving element PD2.

To avoid this drawback, the configuration below is used. As shown in Figure 6B, on a plane orthogonal to the running yarn 1, the opening angle θ3 between the light sources UV1 and UV2 around the running yarn 1 may be set between 90 degrees and 135 degrees. The opening angle θ1 between the light source UV1 and the light receiving element PD2, which receives a reflected light from the light source UV1 and a transmitted light from the light source UV2, may be set at 90 degrees or smaller. The opening angle θ2 between the light source UV2 and the light receiving element PD1, which receives a reflected light from the light source UV2 and a transmitted light from the light source UV1, may be set at 90 degrees or smaller. The light receiving elements PD2 and PD1 may be arranged closer to each other. The light receiving element PD3 may be arranged between the light sources UV1 and UV2 and within the angle (shown by the alternate long and short dash line) between the light receiving elements PD1 and PD2 around the yarn 1. This arrangement and the placement of the diffuser plate 10 enable a flat yarn defect to be detected in all directions using a minimum number of light receiving elements.

For example, if a polypropylene film is mixed into the yarn 1, is flat, and lies at a detected position, for example, with its flat sectional shape extending over the optical axis of the light source UV1, then the light receiving element PD2 receives that part of the ultraviolet light from the light source UV2 which is transmitted through the yarn 1. The light receiving element PD3 receives that part of the ultraviolet light from the light source UV2 which is reflected by the yarn 1. Further, if the polypropylene film is flat and lies at a detected position with its flat sectional shape extending over the optical axis of the light source UV2, then the light receiving element PD1 receives that part of the ultraviolet light from the light source UV1 which is transmitted through the yarn 1. The light receiving element PD3 receives that part of the ultraviolet light from the light source UV1 which is reflected by the yarn 1. It is thus possible to detect a flat portion that may appear at any position within 360 degrees.

In the above embodiments, the white cotton yarn (100%) is used as a material for a spun yarn. However, the present invention is not limited to this aspect. For example, the present invention is applicable to the case in which a spun yarn is generated by mixing polyester, a kind of synthetic fiber, which is cut into equal lengths with cotton (both are white).

Further, in the present invention, the running yarn is irradiated with an ultraviolet ray. Thus, in particular, if the yarn contains white cotton fibers as a material, the white cotton yarn reflects only a small quantity of light and the quantity of light reflected changes significantly in response to the mixture of foreign material in spite of a small impact on the yarn thickness, as shown in Figures 7A and 7B. Accordingly, an accurate device detecting a foreign material defect can be obtained which reliably detects the mixture of homogeneous fibers in various colors and notably heterogeneous white objects such as white polypropylene films simply by detecting the quantity of light reflected.

## Claims

1. A device detecting foreign material in a yarn, **characterized by** comprising floodlighting means for irradiating a running yarn with an ultraviolet ray and reflected light receiving means for receiving that part of the ultraviolet ray applied by said floodlighting means which is reflected by said yarn to output a signal corresponding to the quantity of light received.

2. A device detecting foreign material in a yarn according to Claim 1, **characterized in that** each of said floodlighting means and reflected light receiving means comprises a plurality of floodlighting means for alternately irradiating a running yarn with an ultraviolet ray and two reflected light receiving means each of which receives that part of the ultraviolet ray applied by a corresponding one of said floodlighting means which is reflected by said yarn to output a signal corresponding to the quantity of light received.

3. A device detecting foreign material in a yarn according to Claim 1 or Claim 2, **characterized in that** each of said floodlighting means and reflected light receiving means comprises a plurality of floodlighting means for irradiating a running yarn with an ultraviolet ray, and at least three reflected light receiving means each of which receives that part of the ultraviolet ray applied by a corresponding one of said floodlighting means which is reflected by said yarn, and **in that** foreign material mixed into said yarn is detected on the basis of the quantity of light received by each of said reflected light receiving means.

4. A device detecting foreign material in a yarn according to any one of Claims 1∼3, **characterized by** further comprising transmitted light receiving means receiving that part of the ultraviolet ray applied by the floodlighting means which is transmitted through the yarn to output a signal corresponding to the quantity of light received.

5. A device detecting foreign material in a yarn according to any one of Claims 1∼4, **characterized in that** the light receiving means for receiving the ultraviolet ray includes a visible light cut filter.

6. A device detecting foreign material in a yarn according to any one of Claims 1∼5, **characterized by** comprising an inner wall surface on which the floodlighting means and the light receiving means are arranged to define a space through which the running yarn passes, and an opening through which the yarn can be inserted into said space, and in that a part of said inner wall surface which is opposite said opening is black.
